Europäisches Patentamt

**European Patent Office**

Office européen des brevets

(11) Publication number : **0 401 313 B1**

(12) **EUROPEAN PATENT SPECIFICATION**

(45) Date of publication of patent specification :
**10.05.95 Bulletin 95/19**

(21) Application number : **89908330.7**

(22) Date of filing : **04.01.89**

(86) International application number :
**PCT/US89/00007**

(87) International publication number :
**WO 89/09404 05.10.89 Gazette 89/24**

(51) Int. Cl.[6] : **G01N 33/532,** C12Q 1/68,
G01N 33/53, G01N 33/537,
G01N 33/543, G01N 33/569

(54) **MACROMOLECULAR CONJUGATE.**

(30) Priority : **01.04.88 US 176761**

(43) Date of publication of application :
**12.12.90 Bulletin 90/50**

(45) Publication of the grant of the patent :
**10.05.95 Bulletin 95/19**

(84) Designated Contracting States :
**AT BE CH DE FR GB IT LI LU NL SE**

(56) References cited :
**EP-A- 131 830**
**EP-A- 259 186**
**EP-A- 263 184**
**US-A- 4 719 176**
**CHEMICAL ABSTRACTS, vol. 90, no. 17, 23**
**April 1979, Columbus, OH (US); A. REMBAUM**
**et al., p. 357, no. 136008z**
**BIOLOGICAL ABSTRACTS, vol. 78, no. 5, 1984,**
**Philadelphia, PA (US); E. ANGLES-CANO et**
**al., no. 35637**

(73) Proprietor : **DIGENE DIAGNOSTICS**
**INCORPORATED**
**2301-B Broadbirch Drive**
**Silver Spring, MD 20904 (US)**

(72) Inventor : **TAUB, Floyd, E.**
**10616 Mantz Road**
**Silver Spring, MD 20903 (US)**
Inventor : **ROSHTO, Kelly, K.**
**7304 Hopkins Avenue**
**College Park, MD 20740 (US)**
Inventor : **HIGGS, Thomas, W.**
**225 Alymer Court**
**Westminster**
**Maryland 21157 (US)**

(74) Representative : **Sheard, Andrew Gregory**
**Kilburn & Strode**
**30, John Street**
**London WC1N 2DD (GB)**

## Description

### FIELD OF THE INVENTION

This invention relates to macromolecular conjugates, such as nucleic acid hybridization probes and immunological probes, useful as research and diagnostic tools. More particularly, it relates to making macromolecular conjugates by employing a universal conjugating agent.

### BACKGROUND OF THE INVENTION

Probe technology is emerging as a powerful tool in diagnostic testing, detection of genetic defects and the mapping of prokaryotic and eukaryotic genomes. Nucleic acids are characteristic of and therefore may be used to indicate the presence of a particular genus or species of bacteria or type of virus. They may indicate the presence of genes for pathogenicity or for antibiotic resistance or for a particular genetic disease.

Nucleic acid hybridization probes are well known tools of molecular biology. Grunstein et al., Proc. Nat. Acad. Sci. USA 72:3961 (1975) and Southern, J. Mol.Biol. 98:503 (1975), describe hybridization techniques using radiolabeled nucleic acid probes.

Culture techniques are slow to produce results. Many organisms require incubation over night. Some require incubation for up to a month. Some organisms are difficult to culture.

Monoclonal antibody techniques often have limited specificity. They are subject to undesirable cross reactions and the inability to detect antigenic variants.

Nucleic acid hybridization probes have the advantages over other methods of speed and high specificity. However, existing hybridization techniques which utilize radioisotopes introduce additional expenses in disposal of radioactive waste products and monitoring personnel and the work place for contamination. Autoradiographic detection may require up to two weeks of exposure. These techniques are not suited to commercial areas such as clinical diagnosis.

One technique under development to overcome the drawbacks inherent in radioisotopic probes is the nonradioactive labeling of nucleic acids. Any labeling group that does not impair the ability of the nucleic acid to hybridize with its target may be used to form a probe. After hybridization with a target nucleic acid, the labeled duplex is reacted with additional reagents to provide a signal. The most common method of indirect labeling is to attach biotin, a small vitamin to the nucleic acid, using chemical or enzymatic technique. Following hybridization, the biotin is detected by reaction with avidin, an egg white protein. This reaction can be seen because the avidin has in turn been attached to an enzyme or a fluorochrome. The enzyme can be seen when reacted with certain substrates and fluorochromes can be seen when reacted with ultraviolet light. This method is complex. Therefore, a method attaching a signal-generating enzyme or fluorochrome or other easily visible material directly to the DNA would be advantageous. Antibodies have been employed as bifunctional conjugating agents to link proteins to nucleic acid molecules. U.S. Patent 4,556,643 to Paau et al. discloses the use of antibodies, as well as DNA binding proteins, in hybridization assays. European Patent Application 146,815 discloses hybridization assays which employ antibodies capable of binding to a DNA intercalator molecule. Similar inventions are disclosed in U.S. Patent 4,582,789 to Sheldon and in European Patent Application 131,830. These methods also require complex assay systems as compared to a directly labeled DNA probe.

The ability of biotin to bind to avidin has been exploited in hybridization assays. U.S. Patent 4,581,333 to Kourilsky et al., for example, discloses the use of avidin-bound enzymes to detect hybridization between biotinylated DNA probes and a particular nucleic acid molecule of interest. European Patent Application 133,473 discloses the use of biotin-avidin bridging agents as well as sugar-binding lectins to link the proteins and nucleic acid molecules used in a hybridization assay.

A large number of covalent conjugating agents are known in the immunoassay art, where they have been used to directly attach such labels to antibodies or antigens. Antibodies or antigens labeled in this manner are sometimes referred to herein as immunologized probes. Such agents are also often used to attach immunogens to carriers. Albarella, U.S. Patent 4,469,797, discloses digoxigenin derivatives capable of acting as bifunctional coupling agents to link immunoglobulins to polypeptide carriers. Farina et al., U.S. patent 4,378,428, discloses covalent conjugating agents which may be used in a homogeneous immunoassay. Halmann et al., U.S. Patent 4,302,534, discloses the use of either antigen or antibody labeled with peroxidase in an immunoassay. Devlin, U.S. Patent 3,951,748, discloses an immunoassay in which a coupling agent immobilizes protein molecules to an insoluble matrix. U.S. Patent 3,817,837 to Rubenstein discloses an immunoassay which employs a covalent conjugating agent to bind an enzyme ligand. The use of biotin as a bifunctional conjugating agent is disclosed in U.S. Patent 4,298,685 to Parikh et al. Singh, U.S. Patent 4,241,177, additionally discloses similar covalent conjugating agents.

A small number of proteins bind readily to deoxyribonucleic acid (DNA). These are referred to as DNA binding proteins. Known DNA-binding proteins such as histones, RecA and single-stranded DNA binding protein (SSB) have been employed in hybridization and diagnostic assays. European Patent Applications 183,822 and 164,876 disclose hybridization methods for identifying known genetic sequences in a target DNA molecule which employ DNA binding proteins such as E. coli RecA and SSB. Japanese Patent Application 56001351 discloses a method for quantitatively analyzing DNA binding protein by affinity chromatography using a carrier to which DNA is linked. Histones and some other positively charged proteins form excellent DNA binding proteins. These proteins are candidates for labeling groups. However, most proteins that are signal generating or that would react with signal molecules are not DNA binding proteins. These may be attached to nucleic acids by covalent conjugating agents. Conjugate probes are made by bonding a labeling group to a nucleic acid by using a conjugating agent.

A number of methods are known for covalently crosslinking proteins to nucleic acid molecules. European Patent Application 151,001 discloses a polynucleotide which is covalently crosslinked to a protein molecule. European Patent Application EP 138,357 discloses additional bifunctional covalent conjugating agents. U.S. Patent 4,587,044 to Miller et al. discloses nucleic acids which are modified by esterification with a saturated or unsaturated aliphatic dicarboxylic acid or anhydride to produce a molecule capable of being crosslinked to a protein. Libeskind, U.S. Patent 4,699,876, discloses a number of bifunctional crosslinking agents including N-succinimidyl 4-glyoxalyl-benzoate, carbonyl imidazole, dimethyl superimidate, 1-ethyl,3-dimethylaminopropylcarbodiimide, paranitrophenyl 3-(2-bromo, 3-ketobutylsulfonyl)-propionate or other active esters, glutaraldehyde and other suitable equivalents.

The use of glutaraldehyde as a covalent conjugating agent capable of binding proteins to nucleic acid molecules is disclosed by Borel et al., J. Immunol. Met. 67:289-302 (1984). Borel et al. describe a 2 stage process in which an oligonucleotide was incubated with glutaraldehyde and the oligonucleotide-glutaraldehyde conjugate was then incubated with the desired protein to produce the oligonucleotide-protein conjugate. Glutaraldehyde has also been used to covalently attach histones to nucleic acids as reported by Renz, M., Eur. Mol. Biol. Organ. J. 2:817 (1983). Histones are lysine rich DNA-binding proteins. Glutaraldehyde adds covalent bonds to these two moieties that are already matched by a natural strong affinity. The tight binding between nucleic acids and lysine rich histones allows efficient glutaraldehyde crosslinking at low concentrations.

It is not always possible to find proteins that are easily detectable and also have a strong natural affinity for nucleic acids. Proteins that do not have a natural affinity for nucleic acids will not allow the same efficient glutaraldehyde crosslinking. Therefore, one would expect varying degrees of success from attempts to crosslink a variety of proteins to nucleic acids using glutaraldehyde or any other conjugating agent.

Renz et al., Nucleic Acid Res. 12:3435-3444 (1984) considered ionic binding between the protein and the nucleic acid to be essential to the success of a conjugating agent which covalently binds them. To convert horseradish peroxidase into a DNA-binding protein, polyethylenimine carrying primary amino groups was fused to horseradish peroxidase with p-benzoquinone as the crosslinking agent. This modified horseradish peroxidase had an increased affinity for single stranded DNA and could be covalently conjugated with glutaraldehyde.

A wide variety of cationic detergents possessing hydrophobic groups on one end and positively-charged groups on the other may be prepared. Such detergents are disclosed by, for example, U.S. Patent 4,235,759 (Ohbu) and U.S. Patent 4,454,060 (Lai, K.Y., et al.). Such detergents have found use as cleaning agents such as shampoos, bubble baths and skin cleansers, etc.

Detergents have been utilized in the immunoassay art. Albert, W., et al., for example, in U.S. Patent 4,486,534, teaches the isolation of immunologically active conjugates from a mixture of active and inactive conjugates by putting the mixture in contact with a carrier-bound complex-former such as a fatty acid, and eluting the inactive conjugants with a detergent. Caldwell, in U.S. Patent 4,427,782, teaches the use of an anionic detergent to solubilize an antigen for later purification, and use in the isolation and purification of a corresponding antibody.

Weltman, in U.S. Patent 4,002,532, describes the use of organic polyamine conditioners in connection with the coupling of enzymes to antibodies.

Similar cleanser compositions have been devised which are intended to break natural crosslinks in proteins. (see, for example, US. Patent 4,311,618; Schafer-Burkhara.)

Place et al., J. Immunol. Met. 48:251-260 (1982) discloses a method of improving the adsorption of protein to plastic surfaces to improve the reliability of quantitative solid phase immunoassays. Place et al. observed that antibody to hepatitis B surface antigen (anti-HB$_s$Ag) could be partially removed from a plastic surface by serum albumin. Removal could be substantially prevented if the wells of a polyvinyl chloride (PVC) microtitre plate were first treated with 2% polyglutaraldehyde. Monomeric glutaraldehyde was, in contrast, ineffective to improve the binding of protein to plastic.

Rembaum *et al, J. Immun. Meth.*, **24** (1987), 239-250 disclose the use of a water-insoluble polyglutaraldehyde fraction bound to the surface of polymeric microspheres as probes for binding to various proteins. However, the probes disclosed are, of course, water-insoluble.

## SUMMARY OF THE INVENTION

The present invention provides a new method of forming a water-soluble conjugate probe formed between a macromolecule and a labeling group. The conjugate probe of the present invention is made from virtually any macromolecule by binding to virtually any labeling group.

The present invention also provides hybrids between the conjugate probe and its target nucleic acid.

The present invention also provides a method of detecting the presence of a target nucleic acid by detecting the presence of a labeling group that is bound to a hybrid.

The present invention also provides a test kit for performing hybridization testing comprising the conjugate probe and a hybridization medium.

## BRIEF DESCRIPTION OF THE DRAWINGS

Figure 1 shows a gel electrophoretogram illustrating the conjugation of a nucleic acid to β-phycoerythrin.

Figure 2 shows a gel electrophoretogram illustrating the conjugation of a nucleic acid to horseradish peroxidase.

Figure 3 shows a plot of signal and background of conjugate probes.

Figure 4 shows the results of hybridization between five conjugate probes and four targets.

Figure 5 illustrates a non-linear relationship between the signal intensity and the concentration of conjugation reagents.

Figure 6 shows a gel electrophoretogram illustrating the conjugation of protein pairs with polyglutaraldehyde.

## DETAILED DESCRIPTION OF THE INVENTION

The present invention provides a conjugate probe and a method of making it, a hybrid between target and the probe, a method of detecting the presence of a target molecule by using the probe and a test kit for performing hybridization.

The present invention provides a water-soluble conjugate probe, comprising:

(a) a macromolecule conjugated with

(b) a labelling group through

(c) polyglutaraldehyde.

In order to provide a clear and consistent understanding of the specification and the claims, including the scope given to such terms, the following definitions are provided:

Bridging Reagent: a molecule which acts as a connector between parts of a conjugate or between a conjugate and a solid substance. As used herein, it does not function as a conjugating agent.

Conjugate: a molecular species composed of two or more molecules regardless of the form of attachment. As used herein, a conjugate is comprised of a macromolecule, a labeling group and a conjugating agent.

Conjugating agent: a molecule which forms a bond between two or more other molecules regardless of their relative attraction for each other. As used herein, a conjugating agent is a molecule which forms a stable bond between a macromolecule and a labeling group. The preferred conjugating agent is polyglutaraldehyde.

Complementary conjugate probe: is a probe which binds to its corresponding target. For example, a complementary nucleic acid conjugate probe hybridizes to a complementary target nucleic acid. A complementary antibody conjugate probe binds to its target antigen. A complementary receptor conjugate probe binds to its receptor target molecule.

Complementary nucleic acids: as referred to herein are capable of substantial base pairing throughout their length.

Detergents: are bifunctional molecules having a positively charged region and a hydrophobic region. Detergents are capable of binding to molecules of different types by non-covalent interaction. In this way, detergents are capable of bringing together molecules which have little or no attraction for each other.

Direct labeling: a method of labeling nucleic acids in which the labeling group is attached to the nucleic acid.

Hybridization: a process in which a nucleic acid joins with a complementary nucleic acid through base pairing. The process is sometimes referred to as reannealing.

Labeling group: a molecule which is attached to a biological molecule as a marker. As used herein it is a molecule attached to a macromolecule in a conjugate. Labeling groups may be proteins or non-protein molecules or atoms or groups of atoms which are detectable. Among the labeling groups are radiolabeled molecules, antigens, antibodies, colored or fluorescent dyes, enzymes, chemiluminescent catalysts, phycobilins, ferritin, nucleic acid binding proteins and the like.

Macromolecule: Any large molecule having a 3-dimensional structure. As used herein, it refers to molecule such as a protein or nucleic acid which will attach to a target. It may be attached to a labeling group to form a conjugate probe. The labeling group provides feedback in the form of a detectable molecule or complex.

Monoglutaraldehyde: The singular form of glutaraldehyde. It has been used with limited success as a conjugating agent.

Nucleic acid: a chain of nucleotides. As used herein, nucleic acid refers to any nucleic acid sequence, strand of nucleic acid, oligonucleotide, or polynucleotide formed from the polymerization of nucleotides or the cutting of longer chains of nucleotides. It may be DNA or RNA or any substantially similar polymer. It may be single stranded or double stranded.

Polyglutaraldehyde: A polymer of monoglutaraldehyde having two or more sites at which it may form covalent bonds with other molecules. It has been found to be superior to monoglutaraldehyde as a conjugating agent.

Probe: A nucleic acid or other macromolecule used to detect a target, such as in molecular hybridization to detect complementary nucleic acid in the presence of a large amount of non-complementary nucleic acid or in immunoassays to detect an antigen or an antibody. As used herein, it is referred to as a conjugate probe. A conjugate probe is a probe formed by conjugation of a macromolecule to a labeling group by a conjugating agent. It is detectable by detecting the presence of the labeling group.

Signal molecule: A compound used in the assay of affinity labels as a means of detecting the presence of the labeling group. Some signal molecules are labeling groups which can be detected with a simple assay technique. Examples of signal molecules are fluorochromes and enzymes linked to antibodies.

Substantial sequence homology: Denotes nucleotide sequences that are related enough in sequence to strongly bind to one another. Nucleotide differences between such sequences having substantial sequence homology will be de minimus in affecting the hybridization of the two molecules.

Target: As used herein, the term refers to nucleic acid having sufficient nucleotide base homology with a conjugate probe nucleic acid to permit formation of stable hybrid, an antigen or antibody to which an antibody probe or antigen probe, respectively, will bind or other suitable macromolecule such as a receptor.

One feature of the present invention is a conjugate probe. The conjugate probe comprises at least one macromolecule, a labeling group and polyglutaraldehyde. The macromolecule may be a protein or a nucleic acid. The probe may contain two or more nucleic acids if it is prepared from a mixture of different nucleic acids or if several similar or identified nucleic acids are bound to each other and the label. The conjugate probe may contain a protein and a labeling group. The protein may be, for example, an antigen, an antibody or an immunoglobulin, or a receptor. The macromolecule of the probe may further be a molecule which binds to a receptor. If the probe contains nucleic acid, the conjugate probe functions by hybridizing the macromolecule portion of the conjugate to a complementary portion of a target. If the probe contains an antibody, for example, the conjugate probe functions by binding to the appropriate antigen. The specificity of a conjugate probe requires that not only must the probe be specific to its target, but it must not be functionally altered by its position or chemical association with respect to other molecules forming the conjugate.

The nucleic acid molecule used in one embodiment of the conjugate probe of the present invention may be deoxyribonucleic acid (DNA) or ribonucleic acid (RNA) or a fragment of DNA or RNA or a molecule substantially similar to DNA or RNA such as methylphosphanate nucleic acid or thiolnucleic acid. The DNA or RNA is preferably single-stranded. It may be of eukaryotic, procaryotic or viral origin.

The DNA or RNA may be obtained by using restriction enzymes, chemical synthesis, or sonication. Chemically synthesized nucleic acids can be made such that they have substantial sequence homology to the desired target nucleic acid. Restriction enzymes cut nucleic acids at recognition sites. In the presence of a uniform pool of nucleic acids, restriction enzymes yield a uniform and Predictable heterogenous group of nucleic acids. Sonication applies energy to nucleic acid to overcome chemical bonds. Sonication yields an unpredictable heterogenous group of nucleic acids. Both sonication and restriction enzymes will yield nucleic acids which are homologous to a portion of the nucleic acid from which it was derived. A pool of heterogenous nucleic acids derived from the same virus, for example, will hybridize with its complementary portion of nucleic acid from the same type of virus except where a mutation has occurred. Techniques for the isolation of such nucleic acids are well known to those of ordinary skill in the art. See for example, Maniatis, T., et al. in Molecular Cloning, A Laboratory Manual, Cold Spring Harbor, NY (1982), or Davis, R.W., et al. in A Manual for Genetic Engineering, Advanced Bacterial Genetics, Cold Spring Harbor, NY (1980).

The protein molecule used in a second embodiment of the present invention may be an antigen, an antibody, an immunoglobulin or a receptor. The antibody may be a polyclonal or a monoclonal antibody. These materials are prepared according to conventional techniques. In a third embodiment, the macromolecule may be a molecule which binds to a receptor.

Any labeling group may be used according to the present invention if it will bind to a nucleic acid in the presence of polyglutaraldehyde. For example, any enzyme whose activity may be monitored may be used in accordance with the present invention. It is, however, often preferable to employ enzymes whose presence can be monitored through the use of chromogenic substrates or colorimetric reactions for in situ assays. A stable precipitate results which can easily be seen. Such enzymes include sugar hydrolyzing enzymes such as beta-galactosidase, oxidase enzymes such as glucose oxidase, peroxidase enzymes such as horseradish peroxidase. Light-generating luciferase proteins may be preferable in situ or in other formats.

Other labeling groups may be preferable in other formats. For example, phycobilins are photosynthetic pigments which consist of phycoerythrobilin and phycocyanobilin. They are composed of open tetrapyrroles, covalently bound to two protein molecules. They form aggregates in nature called phycobilisomes which account for the color in red algae and in cyanophytes. Phycobilins are preferred labeling groups because they do not require an enzyme assay for detection. More than one labeling group may be present in the conjugate probe to provide a greater signal. Two or more different labeling groups may be desired for a particular type of assay. Therefore, the labeling groups may be the same or different.

The conjugating agent of the present invention is polyglutaraldehyde. Polyglutaraldehyde can be differentiated from its monomeric form by their absorbance peaks at 235nm and 280nm respectively. Monoglutaraldehyde is believed to undergo aldol condensation above pH 7 to form long-chained $\alpha,\beta$-unsaturated polyglutaraldehyde. The formation of polyglutaraldehyde from glutaraldehyde is known to occur spontaneously and in fact has been reported to seriously degrade the functioning of glutaraldehyde. In order to prevent this degradation, glutaraldehyde is generally supplied bottled under inert gases and refrigerated. The literature indicates that the polymerized contaminant of glutaraldehyde is detrimental for almost all application. For example, PolySciences, August 1986 Data Sheet #124 states that glutaraldehyde is subject to air oxidation and polymerization when in concentrations of 25% or more. The polymeric forms are said to be poor fixatives and inhibit enzymes. Applicants find that, contrary to this teaching, polyglutaraldehyde is a superior conjugating agent and appears to have no inhibitory effect either upon the probe macromolecule or upon the labeling group.

It has now been found that polyglutaraldehyde will form stable water-soluble conjugates between macromolecules and labeling groups. Applicants have shown that polyglutaraldehyde will conjugate: $\beta$-phycoerythrin to $\beta$-phycoerythrin, horseradish peroxidase or DNA; horseradish peroxidase to horseradish peroxidase, glucose oxidase, alkaline phosphatase, avidin or DNA; DNA to glucose oxidase or alkaline phosphatase; and RNA to apoaquarin, urokinase or alcohol oxidase. Thus, it has been found that polyglutaraldehyde is a universal conjugating agent of superior quality. It can conjugate virtually any macromolecule with any labeling group. Proteins bound to labeling groups are useful for example in performing immunoassays. It has also been found that polyglutaraldehyde will form water-soluble conjugates between a nucleic acid and several labeling groups. The resulting conjugate probe yields a strong signal. An example of this effect is seen in the conjugation of several horseradish peroxidase groups with a single nucleic acid using polyglutaraldehyde.

Polyglutaraldehyde is shown to be a superior conjugating agent because it is able to form conjugates between DNA and proteins which monoglutaraldehyde cannot. It is also shown to be capable of forming conjugates with a stronger signal due to the presence of a relatively high number of labeling groups. Polyglutaraldehyde can be used alone or in conjunction with monoglutaraldehyde. The combined uses of these two conjugating agents may be useful, for example, in controlling the average molecular weight of the conjugating agent or for achieving a controlled conjugation reaction.

Polyglutaraldehyde is capable of covalently conjugating virtually any labeling group to any macromolecule by a simple one step incubation process. The process may however be optimized for some conjugates by complexing polyglutaraldehyde (or a mixture of polyglutaraldehyde and monoglutaraldehyde) to either the labeling group or the macromolecule and then incubating the resulting complex with the next member of the conjugate probe. As used herein, the reaction of polyglutaraldehyde with one component of the probe is termed activating the components. Labeling density is achieved by manipulating the mass ratio of labeling group to macromolecule and the concentration of polyglutaraldehyde in the mixture. The mass ratio of the labeling group to macromolecule in the incubation mixture determines the amount of signal that will be detectable from a hybrid. Although mass ratio of 1:2 is detectable, a mass ratio from 2:1 to 15:1 is preferred. Mass ratios from 4:1 to 8:1 are especially preferred. The highest readable signal is obtained at these ratios. The preferred ratio may vary for different signal generators and different assay methods.

Detergents are bifunctional molecules having a positively charged region and a hydrophobic region. They will bind to both nucleic acid and protein by non-covalent interaction. It has now been found that when a detergent is incubated with the substates to be conjugated, covalent conjugation by polyglutaraldehyde is improved.

Polyglutaraldehyde may be used in conjunction with a bridging reagent. The bridging reagent may attach to polyglutaraldehyde which then attaches a macromolecule and a label. For example, a conjugate of the sequence macromolecule-polyglutaraldehyde - bridging reagent-polyglutaraldehyde-label may be formed. A sequence may also be formed in which the bridging reagent binds directly to either the macromolecule or the labeling group. The bridging reagent may be albumin, polylysine or any molecule with at least one group that is reactive with an $NH_2$ group and another group that is reactive with a labeling group. The bridging reagent may be used in conjunction with any solid support such as a filter, a column or a microtitre plate.

Hybrids are made by allowing the macromolecule of the conjugate probe to pair with its target. This may be done in a variety of ways. The target macromolecule may be bound to a solid support such as an affinity column. The conjugate probe is allowed to pass across the column and bind with its target. A viral, body fluid, tissue, or cell sample suspected of containing target nucleic acid or antigen may be placed on an inert solid support. In some cases it may be desirable to amplify the sample in culture before applying a conjugate probe. If necessary, the sample is then treated with a reagent effective in opening viral capsids, cells and tissues and denaturing the nucleic acid. This may be done before, during, or after the sample is fixed to an inert solid support. Several reagents may be used to denature nucleic acid such as alcohols, amides, ureas, phenols, sulfoxides.

Sandwich hybridization may be performed by hybridizing a chemically biotinylated DNA probe to a conjugate probe having homologous DNA and being conjugated by polyglutaraldehyde. The hybrids can be isolated by reacting them with avidin that is immobilized on a microtitre well. Another form of sandwich hybridization is performed by hybridizing a chemically biotinylated DNA probe and a non-homologous conjugate probe made using polyglutaraldehyde to target DNA that will hybridize to both probes. The hybrids can be isolated by reacting the hybridization mixture with immobilized avidin. These and other methods of preparing samples will occur to one of ordinary skill in the art. Sandwich hybridization of antigens and antibodies are also well known in the art.

Hybridization occurs easily in fixed samples in which the nucleic acid has been denatured. The sample is reacted with a conjugate probe. Nucleic acid of the conjugate probe is allowed sufficient time to locate and hybridize to its target. Usually 10 minutes to two hours are allowed. Non-hybridized conjugate probe is washed from the sample. A concentration of from 0.3 to 30.0μg/ml of conjugate probe is preferred when applying it to a fixed sample of denatured nucleic acid.

The best mode of practicing the present invention would have a high signal to background ratio. Conjugate probes at a concentration of 2.5μg/ml were applied to samples containing target. The signal and background were measured as the mass ratio of horseradish peroxidase to DNA in a conjugate probe increases from 1:2 to 8:1. Two effects are evident. The amount of signal increased until the mass ratio reached 4:1 but did not further increase as the mass ratio of protein to DNA increased above 4:1. The level of background remained at zero until the mass ratio reached 4:1 and increased above zero as the ratio increased above 4:1. An ideal conjugate probe gives a strong signal without background. However, low levels of background are easily tolerable in the presence of a strong signal.

The concentration of reagents in the conjugation mixture can determine the intensity that arises from a hybridized conjugate probe. A final concentration of reagents greater than 0.10 mg/ml in conjugate probe mixtures having a labeling group to macromolecule, such as nucleic acid, mass ratio of 4:1 and a polyglutaraldehyde concentration of 2.5% is preferred. A concentration of reagents greater than 0.25 mg/ml is especially preferred.

Extent and specificity of nucleic acid hybridization is affected by a number of factors. For example, a covalent bond between the conjugating agent and a nucleic acid or between the nucleic acid and a labeling group could alter the specificity of the nucleic acid for its target. The purity of the nucleic acid preparation in the conjugate probe will affect specificity. The thermal stability of the probe nucleic acid is increased in proportion to the relative number of G-C units present. Specificity can be reduced by breakdown of the nucleic acid at high temperatures. Hybridization typically proceeds at temperatures between 37° and 70°C.

Specificity decreases as the length of the nucleic acid decreases. Little or no specificity can be assigned to hybridizations involving short nucleic acids (below 12 nucleotides). Nucleic acids having 100 or more bases are preferred. The ionic strength of the hybridization solution affects the rate of hybridization and also the thermal stability of resulting hybrids. Conjugate probe concentration should be in excess, preferably about 100 fold or more of target nucleic acid to promote hybridization. Incubation time must be sufficient to allow sufficient hybridization for detection. Volume exclusion reagents such as dextrin effectively increase the concentration of the conjugate probe thereby increasing the rate of hybridization. Conjugate probes made with polyglutaraldehyde have been found to exhibit 100% specificity for target nucleic acid by hybridizing to target nucleic acid and failing to hybridize to non-target nucleic acid in every case tested.

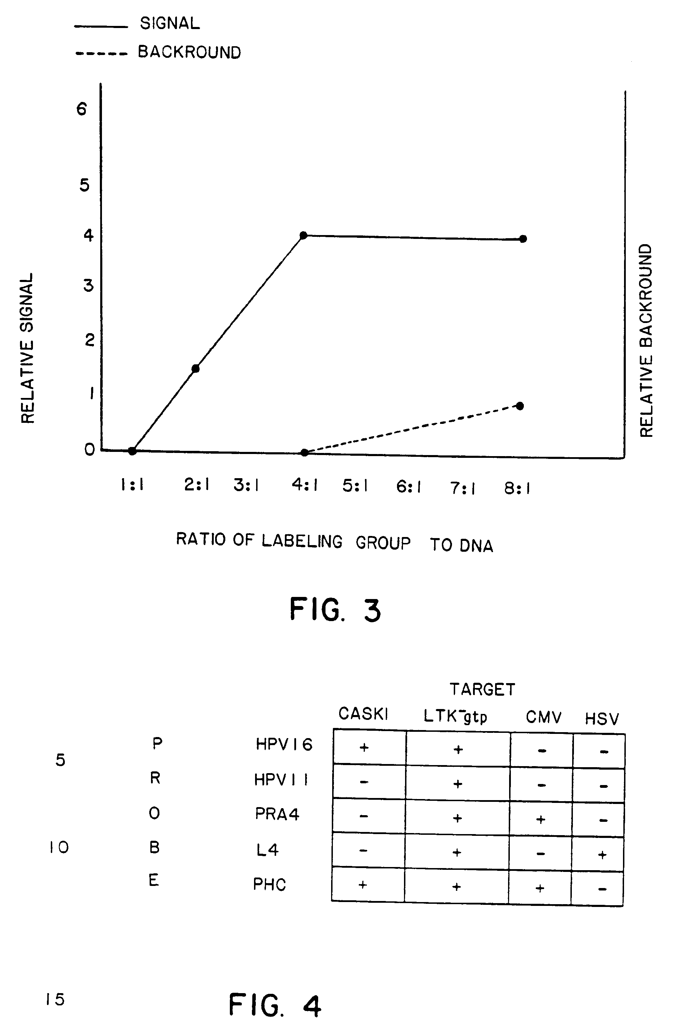

The specificity of nucleic acid in a conjugate probe for its target should not be significantly altered by the chemical bonds formed during conjugation. Figure 4 shows the results of five (5) conjugate probes made with polyglutaraldehyde when tested against four (4) targets. In each case the conjugate probe hybridized to target DNA and failed to hybridize to non-target DNA.

Target nucleic acid is detected by detecting the presence of the labeling group on the conjugate probe. After hybridization, a washing step removes any conjugate probe that is not hybridized. A labeling group is usually detected by its reaction with a signal molecule. Among the variety of detection means are spectroscopic, Photochemical, immunochemical, biochemical, chemical, fluorescence, luminescence and isotopic. A labeling group may be detected directly. Directly detectable labeling groups include, for example, radioisotopes, fluorochromes and phycobilins. It is preferred not to use a radioisotope in order to avoid its disadvantages. A labeling group may be detected indirectly as a result of a chemical reaction which forms a detectable product. For example, an enzyme might react with a signal molecule to form a detectable precipitate. Examples of signal molecules are enzyme substrates, chromogens and prechromogens.

The present invention provides a test kit for detecting the presence of target macromolecule in a sample. By the term "kit" is meant a packaged combination of one or more containers, devices or the like holding the necessary reagent elements for detecting the presence of at least one target macromolecule. It usually includes written instructions for performing assays. In all cases, the kit must contain one or more conjugate probes according to the number of targets sought and the format of the assay. (The sandwich format assay requires more than one conjugate probe). The labeling group may be the same or different for each conjugate probe. The kit for detecting a target nucleic acid may also contain (1) a denaturation agent for converting double stranded target nucleic acid to single stranded nucleic acid in a sample; (2) A lysing agent for treating the sample to release nucleic acid or to allow entry of the conjugate probe; and (3) A hybridization solution for maintaining optimal conditions to allow for specific hybridization. Optionally, the kit may also contain a solid support for immobilizing either the sample or the target nucleic acid from the sample. Alternatively, the conjugate probe can be immobilized on the support. Where it is required for detection of the labeling group, a signal molecule may also be provided. Appropriate washing buffer to maintain stringency and specificity may also be provided.

EXAMPLE 1

β-phycoerythrin-DNA Conjugates

In this example the ability of glutaraldehyde and polyglutaraldehyde to conjugate single stranded DNA to β-phycoerythrin was compared. This example demonstrates that polyglutaraldehyde is able to conjugate β-phycoerythrin to DNA. However, glutaraldehyde is ineffective at conjugating β-phycoerythrin to DNA.

The following mixtures were prepared and incubated: DNA with β-phycoerythrin, β-phycoerythrin with β-phycoerythrin and DNA with DNA.

| | A | B |
|---|---|---|
| β-phycoerythrin (20 $\mu$g) | 11.7 $\mu$l | 11.7 $\mu$l |
| glutaraldehyde (10%) | 2 $\mu$l (poly) | 2 $\mu$l (mono) |
| single stranded DNA (10 $\mu$g) | 6.7 $\mu$l | 6.7 $\mu$l |
| $H_2O$ | 0 $\mu$l | 0 $\mu$l |

| | C | D |
|---|---|---|
| β-phycoerythrin (20 $\mu$g) | 11.7 $\mu$l | 0 $\mu$l |
| glutaraldehyde (10%) | 2 $\mu$l (poly) | 2 $\mu$l (mono) |
| single stranded DNA (10 $\mu$g) | 0 $\mu$l | 6.7 $\mu$l |
| $H_2O$ | 6.7 $\mu$l | 11.7 $\mu$l |

|  | E | F |
|---|---|---|
| β-phycoerythrin (20 µg) | 0 µl | 11.7 µl |
| glutaraldehyde (10%) | 2 µl (poly) | 2 µl (mono) |
| single stranded DNA (10 µg) | 6.7 µl | 0 µl |
| $H_2O$ | 11.7 µl | 6.7 µl |

The final concentration of conjugating agent in each mixture was 1.0%. The mixtures were incubated at 37°C for one hour and then cooled in an ice bath until analyzed by electrophoresis.

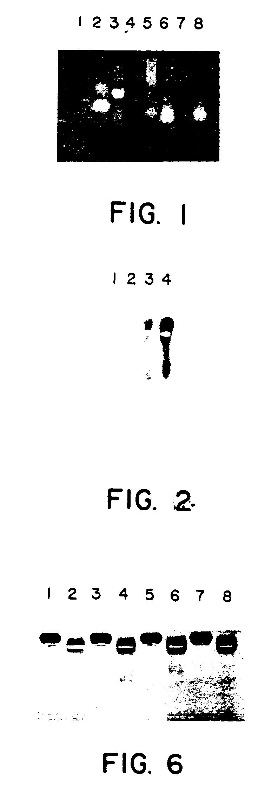

Electrophoresis was carried out on eight samples in adjacent wells on an agarose gel as shown in Figure 1 on samples of:

(1) DNA (control)
(2) β-phycoerythrin (control)
(3) polyglutaraldehyde with DNA and β-phycoerythrin
(4) polyglutaraldehyde with DNA, without β-phycoerythrin
(5) polyglutaraldehyde with β-phycoerythrin, without DNA
(6) glutaraldehyde with DNA and β-phycoerythrin
(7) glutaraldehyde with DNA without β-phycoerythrin
(8) glutaraldehyde with β-phycoerythrin without DNA

β-phycoerythrin fluoresces red and can be easily seen without staining. The results are shown in Figure 1. Samples 1 and 2 are standard controls which indicate the migration patterns of DNA and β-phycoerythrin respectively under the influence of an electric current in an agarose gel. Samples 3, 4, and 5 contain polyglutaraldehyde. Sample 3 shows a delayed migration pattern relative to β-phycoerythrin and thus indicates the presence of both β-phycoerythrin and of DNA. In order to confirm this, the electrophoresis gel was then stained with toluidine blue to show the presence of DNA. The blue color indicating the presence of DNA appeared exactly superimposed with this band confirming it contains both β-phycoerythrin and DNA. This indicates that conjugation occurred between DNA and β-phycoerythrin in the presence of polyglutaraldehyde.

Sample 4 shows that polyglutaraldehyde is unreactive in crosslinking DNA with DNA. However, Sample 5 illustrates the effectiveness of polyglutaraldehyde in crosslinking proteins to proteins. Since no DNA is present but the migration of the BPE into the gel is prevented.

Samples 6, 7, and 8 contain glutaraldehyde. Samples 6 and 8 show a migration pattern typical of β-phycoerythrin. Sample 7 shows a pattern identical to Sample 1. Taken together, Samples 6, 7, and 8 indicate that conjugation in the presence of glutaraldehyde did not occur.

EXAMPLE 2

Horseradish Peroxidase-DNA Conjugates

In this Example, the ability of polyglutaraldehyde and glutaraldehyde to conjugate single stranded DNA to horseradish peroxidase was evaluated. This example shows that polyglutaraldehyde can conjugate large amounts of horseradish peroxidase to DNA.

The following mixtures were prepared in ratios of labeling group to DNA of 1:1, 2:1, 4:1 and 8:1, respectively.

1:1

1µl 10mg/ml/horseradish peroxidase
2.5µl polyglutaraldehyde (10%)
4.16µl single stranded DNA (2.4mg/ml)
2.34µl $H_2O$

2:1

    1μl 20mg/ml horseradish peroxidase
    2.5μl polyglutaraldehye (10%)
    4.16μl single stranded DNA (2.4mg/ml)
    2.34 $H_2O$

4:1

    2μl 20mg/ml horseradish peroxidase
    2.5μl polyglutaraldehyde (10%)
    4.16μl single stranded DNA (2.4mg/ml)
    1.34μl $H_2O$

8:1

    4μl 20mg/ml horseradish peroxidase
    2.5μl polyglutaraldehyde (10%)
    4.16μl single stranded DNA (2.4mg/ml)
    0μl $H_2O$

The mixtures were incubated at 37°C for 45 minutes and then cooled in an ice bath until analyzed by electrophoresis. The final concentration of conjugating agent in each mixture was 2.5%.

Electrophoresis was carried out on the samples in adjacent wells on an agarose gel as shown in Figure 2. From left to right the samples were as follows. They contain horseradish peroxidase to DNA ratios of 1:1, 2:1, 4:1 and 8:1, respectively.

The electrophoresis gel was stained with nickel enhanced diaminobenzidine/$H_2O_2$. Horseradish peroxidase is detected by deposition of a black precipitate. DNA is below detectable levels.

The presence of horseradish peroxidase is indicated by the blackened portions of the gel. The presence of higher molecular weight conjugates is indicated by slow migration relative to lower molecular weight conjugates. In samples 1, 2, 3, and 4, the pattern of migration indicates that the molecular weight of the conjugate increased as the ratio of horseradish peroxidase to DNA increased. In each successive sample, the pattern of migration is shorter.

EXAMPLE 3

Signal Analysis of Probes

For conjugate probes that hybridize in situ it is ideal to obtain a strong, easily detectable signal and no background noise that might obscure the signal. The greater the quantity of detectable protein in the probe, the stronger the signal. This example shows that polyglutaraldehyde conjugate probes having a horseradish peroxidase to DNA ratio of 4:1 gave the highest detectable signal. The probes having an 8:1 ratio gave an equivalent signal to those having a 4:1 ratio. When the signal and background noise of conjugate probes having a 4:1 ratio were measured at 2.5μg/ml. 2.5μg/ml was found to give the highest signal without measurable background noise.

Conjugate probes composed of horseradish peroxidase, DNA and 2.5% polyglutaraldehyde were prepared as described in Example 2 and hybridized to test cells in situ. Samples having horseradish peroxidase to DNA ratios of 1:2, 1:1, 2:1, 4:1, and 8:1 were prepared. Eukaryotic target cells for which the probe DNA was specific were fixed to glass slides by a standard ethanol procedure. Signal and background noise were measured by microscopic observation.

When the signal from conjugate probes having different horseradish peroxidase to DNA mass ratios were compared, the signal increased from 1:2 to 4:1 and leveled off. The amount of signal at 4:1 and at 8:1 was equivalent. Background noise was measured through the same range of mass ratios. Background noise was effectively zero between 1:2 and 4:1, but increased above zero between 4:1 and 8:1. Figure 3 plots the signal and noise levels at increasing ratios for probe concentration of 2.5μg/ml.

## EXAMPLE 4

Specificity Analysis of Conjugate Probes Made With Polyglutaraldehyde

It is well known that covalent bonding between molecules alters their local properties. The specificity of nucleic acid probes depends upon the attraction between the nucleic acid moiety and its target nucleic acid. The specificity of polyglutaraldehyde conjugated probes was tested by hybridizing the probes to the DNA of cells known to contain DNA complementary to the probe nucleic acids.

The probe DNA was shown to have specificity by hybridizing with its intended target in every instance and failing to hybridize with non-target DNA in every instance. Therefore, polyglutaraldehyde does not reduce the specificity of conjugate probes for their targets.

Conjugate probes were prepared by sonicating plasmids containing DNA of a specific virus. The following mixture prepared and incubated to make conjugate probes having a labeling group to DNA ratio of 4:1. Target cells were fixed on microscope slides by an alcohol procedure. Conjugate probes having a solution concentration of $25\mu g/ml$ were applied to target cells.

Probes containing Human Papilloma virus #16 (HPV16), Human Papilloma virus #11 (HPV11), Cytomegalo virus (PRA4), Herpes virus (4L) and Human specific sequence (PHC), respectively, were reacted with Caski and $LTK^-gpt$ cells and also with cytomegalovirus (CMV) and herpes simplex virus (HSV) containing cells.

Caski cells are a transformed human cell line that contains integrated HPV16 sequence. A probe containing either HPV16 DNA or PHC DNA would be expected to hybridize with DNA in the Caski cell. $LTK^-gpt$ cells contain DNA present in each probe plasmid. Thus they should react with all probes.

Each of the conjugate probes tested would be expected to hybridize to its respective homologous DNA target. Figure 4 shows the results of hybridization. In each hybridization experiment the probe used reacted with its expected target and failed to react with the control.

## EXAMPLE 5

The Analysis of Glucose Oxidase-DNA Conjugates by Sandwich Hybridization

The presence of glucose oxidase-DNA conjugate probes made with polyglutaraldehyde was determined by sandwich hybridization. The system of sandwich hybridization used consists of hybridizing a chemically biotinylated DNA probe to a complementary glucose oxidase-DNA probe synthesized using polyglutaraldehyde. The hybrids were isolated by reacting the hybridization mixture with avidin that had been immobilized on a microtitre well. After washing, only the unreacted biotinylated DNA and biotinylated DNA hybridized to the complementary glucose oxidase DNA remained in the well. The well was then reacted with a signal reagent that turns blue in the presence of glucose oxidase but is unaffected by biotin labeled DNA. Hence, a blue color would indicate that glucose oxidase was bound to a DNA which hybridized to a DNA containing biotin. If glucose oxidase was not bound to the DNA probe or if that probe could not hybridize to the biotinylated target, no enzyme and (thus no color) would remain in the well following washing.

A conjugation mixture was prepared by adding:

$22\mu l$ single-stranded DNA $(20\mu g)$;

$1.0\mu l$ glucose oxidase $(40\mu g)$;

$3.0\mu l$ 10% polyglutaraldehyde: $4.0\mu l$ $H_2O$.

This mixture was incubated at 37°C for one hour and kept on ice.

Hybridization

$1.0\mu l$ of glucose oxidase-DNA conjugate probe (667ng) was hybridized to 460ng of single-seranded biotinylated DNA in 1.2x SSC (0.2M Na+); 0.6mg/ml yeast RNA, 5.0% polyethylene glycol in $50\mu l$ total volume at 50°C for one hour, allowed to cool to room temperature, then placed on ice.

Coating of Microtitre Wells with Avidin/Collection of Hybrids

$50\mu l$ of 0.1mg/ml avidin in 50mM sodium bicarbonate was placed on the bottom of each well of a 96-well microtiter plate and allowed to sit undisturbed at room temperature for 90 minutes. The avidin was removed, and each well was rinsed 10 times with 1 x phosphate buffered saline (PBS, 7mM $PO_4$, pH 7.2, 15mM NaCl) and blocked with $100\mu l$ of 3% Bovine Serum Albumin (BSA) 0.4M NaCl 0.1M sodium bicarbonate. After 60 minutes of blocking, $65\mu l$ of the BSA solution was removed and $5\mu l$ of hybridization mix was added. At room temperature, the plate was shaken for 30 minutes, rinsed 10 times with a 0.6x solution of sodium saline citrate (SSC 90mM NaCl and 9.0mM sodium citrate) pH 7.2 washed 3 times for 10 minutes, each time with 0.6x SSC, 0.2% sodium dodecyl sulfate (SDS), and re-rinsed 10 times with 0.6x SSC.

Colorimetric Determination of Hybridization

Each well was reacted for 3.5 minutes with 200μl of glucose oxidase reagent containing 0.6μg/ml horse-radish peroxidase, 10% B-D glucose, 0.1mg/ml tetramethylbenzidine in dimethy sulfoxide (DMSO), 50mM NaAc pH 5.0. The reaction was stopped with 100μl 2.0M $H_2SO_4$, and the optical density (OD) at 450nm was recorded spectrophotometrically.

## Results

| Probe | Target | $OD_{450}$ |
|---|---|---|
| DNA-glucose oxidase | complementary biotinylated DNA | 0.669 |
| DNA-glucose oxidase | non-complementary biotinylated DNA | 0.106 |

Thus, significant color was seen only when a conjugate probe complementary to a biotin labeled target was present.

## EXAMPLE 6

Analysis of Alkaline Phosphatase-DNA Conjugates by Sandwich Hybridization

The presence of alkaline phosphatase-DNA conjugates made with polyglutaraldehyde was determined by sandwich hybridization of two non-homologous probes to target DNA.

The system of sandwich hybridization used involved hybridizing a chemically biotinylated DNA conjugate probe and an alkaline phosphatase DNA probe to target DNA that will hybridize to both probes such that after hybridization the two probes will be linked via the target. The two probes used in this system have little or no homology to each other. The hybrids were isolated by avidin-biotin binding as described in the previous example and were reacted overnight with alkaline phosphatase reagent (15mM p-nitrophenylphosphate). The presence of yellow color indicated the presence of the hybrid described above.

The amount of hybridized alkaline phosphatase-DNA probe in each sample was indicated by the optical density of yellow color. A background color density was measured in a control sample containing no target DNA.

A conjugation mixture was prepared by adding:

6.7μl single-stranded DNA (10μg);
10.0μl alkaline phosphatase (20μg);
5.0μl 1.0% polyglutaraldehyde; and
28.3μl $H_2O$.

This mixture was incubated at 37°C for 30 minutes, then placed on ice.

Hybridization

Hybridizations were carried out using 200pg/μl alkaline phosphatase-DNA conjugate probe, 200pg/μl biotinylated-DNA probe, 1.2x SSC (0.2M Na+), 0.1% SDS, 0.6mg/ml yeast RNA, 5.0% polyethylene glycol, 17mM Tris pH 8.8, 1.7mM $MgCl_2$, 4.15mM $(NH_4)_2SO_4$, 1.7μM ethylene diamine tetraacetic acid (EDTA), 0.25% DMSO. Various concentrations of target DNA were hybridized in the above solution for two hours at 50°C in 50μl total volume.

Coating of Wells with Avidin/Collection of Hybrids

250μl of 0.1mg/ml avidin was placed in each well of a 24-well microtiter dish and allowed to sit undisturbed at room temperature for 2.5 hours. The avidin was removed, and each well was rinsed 10 times with 1.0x PBS and blocked for one hour with 500μl of 3.0% BSA 0.4M NaCl 0.1M sodium bicarbonate. After blocking, 300μl

of the BSA solution was removed, and the entire 50μl of hybridization mix was added to the well. The plate was reacted and washed as described in Example 5.

Colorimetric Determination of Hybridization

Each well was reacted with 300μl of 15mM p-nitrophenylphosphate in 1.0M diethanolamine, 1.0mM $MgCl_2$ pH 9.8 overnight at room temperature. The OD at 405nm of the solutions were recorded spectrophotometrically.

## Results

| Character of Target | Amount of Target | $OD_{405}$ |
|---|---|---|
| Hybridizable | 70pg | 0.489 |
| | 20pg | 0.218 |
| | 10pg | 0.142 |
| control | --- | 0.098 |

EXAMPLE 7

The Ability of Urokinase, Apoaquorin, and Alcohol Oxidase to Conjugate to to PolyA, Using Polyglutaraldehyde

Polyribonucleic acid (PolyA) was used to demonstrate that conjugates of nucleic acid and virtually any protein can be formed using polyglutaraldehyde. Affinity chromatography on oligo-dT-cellulose readily separates unconjugated protein from polyA-protein conjugates.

The results of this example show that polyglutaraldehyde will conjugate randomly chosen proteins to nucleic acid.

Terminology

1. Conjugation mixture = poly-A + protein + Polyglutaraldehyde
2. Mixture = poly-A + protein with no polyglutaraldehyde
3. Mock conjugation mixture = protein + polyglutaraldehyde with no poly-A

Conjugation mixtures were prepared as follows:

A)
35μl urokinase (17μg)
5μl 1% polyglutaraldehyde
10μl RNA (25μg)
0μl $H_2O$

B)
3.16μl apoaquorin (50 μg)
5μl 10% polyglutaraldehyde
10μl RNA (25μg)
31.4μl $H_2O$

C)
1μl alcohol oxidase (50μg)
5μl 1% polyglutaraldehyde
10μl RNA (25μg)
34μl $H_2O$

Each mixture was incubated for one hour at 37°C.

Analysis of Conjugates by Oligo-dT Cellulose Chromatography

About 0.1g of oligo-dT cellulose (Collaborative Research) was equilibrated to 20mM sodium citrate, 0.4M NaCl, pH7.2. The conjugate mix was adjusted to the same salt concentration, applied to the top of the column and allowed to adsorb onto the resin. The column was then washed with about 10ml of the above buffer, saving the first two 1.5ml fractions. These wash fractions contained unconjugated protein and excess polyglutar-aldehyde. The column was then eluted with 10ml of distilled water, again saving the first two 1.5ml fractions. These elution fractions contained unconjugated polyA and polyA-protein conjugate.

Results

Analysis of fractions was performed by measuring the OD at 260nm and by Biorad Protein Assay. 400μl of each saved fraction was reacted with 100μl Biorad Protein Assay solution for 10 minutes, and the OD at 595nm was recorded spectrophotometrically.

$$\% \text{ protein conjugated} = \frac{OD_{595} \text{ elution fraction \#1}}{OD_{595} \text{ elution fraction \# 1} + OD_{595} \text{ wash fraction \#1}}$$

| Sample | % Protein Conjugated |
|---|---|
| polyA apoaquorin conjugate | 99% |
| polyA apoaquorin mixture | 0% |
| polyA apoaquorin mock conjugate | 0% |
| polyA urokinase conjugate | 83% |
| polyA urokinase mixture | 0% |
| polyA urokinase mock conjugate | 0% |
| polyA alcohol oxidase conjugate | 32% |
| polyA alcohol oxidase mixture | 0% |
| polyA alcohol oxidase mock conjugate | 0% |

Even though the percentage of protein conjugated varies, each protein is conjugated with polyglutaraldehyde. The conjugation procedure for each individual protein can be optimized to yield higher amounts of protein bound in the conjugate.

Example 8

Conjugation of Avidin to Horseradish Peroxidase

The wells of a 96-well microtitre plate were coated with a 50.0μl solution of avidin at a concentration of 0.1mg/ml in a solution of 50.0mM sodium bicarbonate (BSA) pH8.1 for one hour. The solution was removed from the wells. The wells were rinsed ten times with lx (PBS), and blocked with 200μl of blocking solution (3% BSA, 0.4M NaCl, 0.1M sodium bicarbonate) for 30 minutes.

145μl of blocking solution was removed from 1/2 of the wells and 5.0μl of a solution of single-stranded biotinylated DNA at a concentration of 400pg/μl was added. The plate was shaken for 30 minutes, washed ten times with 1x PBS, and reblocked with 200μl of blocking solution for 30 minutes.

145μl of blocking solution was removed from all of the wells and 5.0μl of avidin-horseradish peroxidase suspected conjugate in blocking solution at concentrations of 100ng/μl, 20ng/μl, 4mg/μl, 0.8ng/μl was added to selected wells. The wells were shaken for 30 minutes and then washed ten times with lx PBS solution.

Each well was reacted with 200μl tetramethylbenzidine (TMB) for 20 minutes. The reaction was stopped with 2.0M $H_2SO_4$. An optical density reading at 450nm was recorded.

## Results

### A. Avidin Wells Reacted with 2ng Single-Stranded Biotinylated DNA

$OD_{450}$ for Amount of Avidin + Horseradish Peroxidase Applied to Each Well

Conjugate:   Avidin + Horseradish Peroxidase + Polyglutaraldehyde

| 500ng | 100ng | 20ng | 4ng | 0.8ng |
|---|---|---|---|---|
| 0.175 | 0.125 | 0.084 | 0.037 | 0.033 |

Conjugate:   Avidin + Horseradish Peroxidase + no Polyglutaraldehyde

| 500ng | 100ng | 20ng | 4ng | 0.8ng |
|---|---|---|---|---|
| 0.019 | 0.020 | 0.023 | 0.024 | 0.023 |

### B. Avidin Wells not Reacted with Biotinylated DNA $OD_{450}$ for Amount of Avidin + Horseradish Peroxidase Applied to Each Well

Conjugate:   Avidin + Horseradish Peroxidase + Polyglutaraldehyde

| 500ng | 100ng | 20ng | 4ng | 0.8ng |
|---|---|---|---|---|
| 0.026 | 0.029 | 0.018 | 0.015 | 0.032 |

Conjugate:   Avidin + Horseradish Peroxidase + no Polyglutaraldehyde

| 500ng | 100ng | 20ng | 4ng | 0.8ng |
|---|---|---|---|---|
| 0.018 | 0.022 | 0.023 | 0.034 | 0.055 |

Conclusions

This example shows that conjugation of horseradish peroxidase to avidin occurs only in the presence of polyglutaraldehyde (Results A), and that neither avidin + horseradish peroxidase conjugate or mixture bind nonspecifically to an avidin-coated well. We can detect 20ng of avidin + horseradish peroxidase conjugate by this method.

Example 9

Determination Of Minimum Concentration For Making Conjugate Probe

Conjugate probes were made using DNA from human specific sequence (PHC) and cytomegalovirus (PRA4) with horseradish peroxidase at a mass ratio of 4:1 of enzyme to DNA. The conjugation mixture contained 2.5% polyglutaraldehyde. The mixture was incubated for 45 minutes at 37°C in buffer solution at pH 6.5-7.0. Each conjugate probe was mixed to final reagent concentrations of 1.0mg/ml, 0.5mg/ml, 0.25mg/ml and 0.10mg/ml before dilution. The quality of conjugates made at these concentrations was compared by measuring the signal from hybrids formed from these conjugates with target DNA.

Each conjugate probe solution was diluted in hybridization buffer to 10μl/ml to make a hybridization mixture. Hybridization solution (25μl) was applied to microscope slides with wells containing LTK⁻gpt and CMV target cells. Hybridization took place for 20-60 minutes at 37°C in a humid chamber. The wells were washed for 10 minutes, stained with DAB for 15 minutes, and counterstained with nuclear fast red.

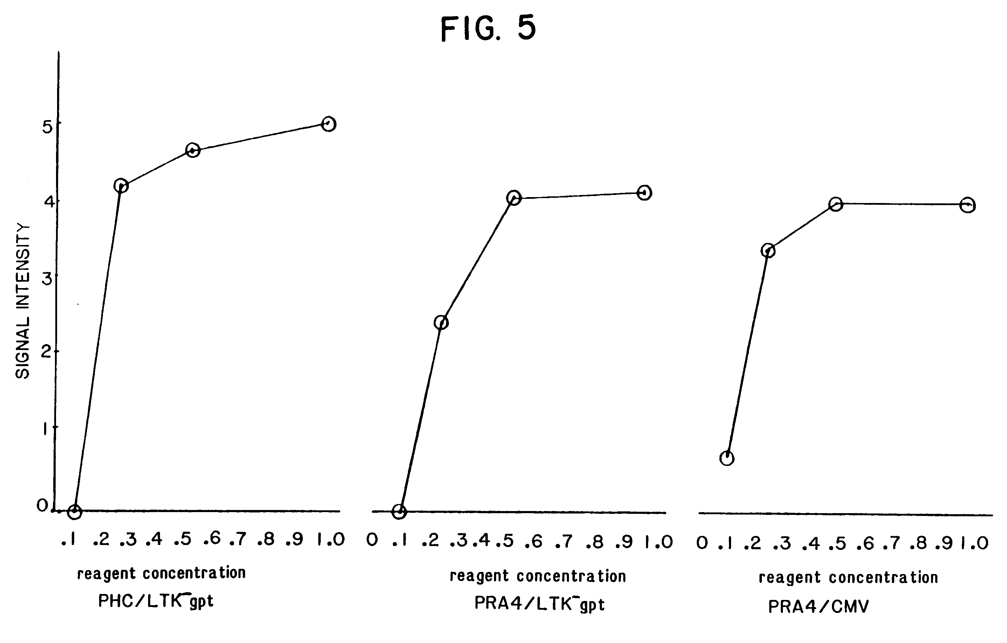

Hybrids from conjugate probes made at 1.0mg/ml, 0.5mg/ml and 0.25mg/ml produced excellent signal intensity. The signal intensity from conjugate probe made at 0.1mg/ml was greatly diminished. Figure 5 illustrates a non-linear relationship between the signal intensity and the change in concentration of conjugation reagents in the incubation solution between 1.0mg/ml and 0.1mg/ml. For each type of conjugate probe a dramatic and unexpected drop in signal intensity occurs between 0.25mg/ml and 0.1mg/ml.

Conclusion

The signal intensity of a conjugate probe is affected by the reagent concentration at the time the conjugate probe is made. Effective conjugation occurs at reagent concentrations above 0.1mg/ml.

Example 10

To demonstrate the ability of polyglutaraldehyde to form conjugates between proteins, a variety of protein pairs were incubated with polyglutaraldehyde and the results determined by electrophoretic analysis on an agarose gel.

(A) Preparation of Conjugates

All conjugates were prepared in a concentration of 1mg/ml of reagent. All solutions were incubated for 60 minutes at 37°C. The following proteins were paired in the conjugation solution:

(1) Horseradish peroxidase + Horseradish peroxidase
(2) Horseradish peroxidase + glucose oxidase
(3) Horseradish peroxidase + alkaline phosphatase
(4) Horseradish peroxidase + β-phycoerythrin
(5) β-phycoerythrin + β-phycoerythrin

Samples were prepared with and without polyglutaraldehyde.

Incubated samples were subjected to electrophoretic analysis on an agarose gel. Figure 6 illustrates the migration pattern of the protein pairs. Each pair contains horseradish peroxidase and a second protein. The table below indicates the second protein and the presence or absence of a conjugating agent are indicated according to the well from which it migrated:

| Well | Protein | Polyglutaraldehyde |
|------|---------|--------------------|
| (1) | Horseradish peroxidase | - |
| (2) | Horseradish peroxidase | + |
| (3) | Glucose oxidase | - |
| (4) | Glucose oxidase | + |
| (5) | Alkaline phosphatase | - |
| (6) | Alkaline phosphatase | + |
| (7) | β-phycoerythrin | - |
| (8) | β-phycoerythrin | + |

Horseradish peroxidase is a positively charged protein. Glucose oxidase, alkaline phosphatase, and β-phycoerythrin are negatively charged proteins. Conjugates would be expected to have a net negative charge, a net positive charge, or be too large to migrate in the gel.

Migration patterns showed that each sample incubated with polyglutaraldehyde formed conjugates. However, samples incubated in the absence of polyglutaraldehyde exhibited the migration patterns of the unconjugated horseradish peroxidase.

**Claims**

1. A water-soluble conjugate probe, comprising:
   (a) a macromolecule conjugated with
   (b) a labelling group through
   (c) polyglutaraldehyde.

2. A conjugate probe as claimed in claim 1, further comprising a bridging reagent.

3. A conjugate probe as claimed in claim 1 wherein the macromolecule is a protein (such as an antigen, an antibody, an immunoglobulin or a receptor), a nucleic acid or a receptor target molecule.

4. A water-soluble conjugate probe comprising:
   (a) nucleic acid conjugated with
   (b) a labelling group through
   (c) polyglutaraldehyde.

5. A conjugate probe as claimed in claim 4 wherein the nucleic acid is DNA, RNA or a similar polymer.

6. A conjugate probe as claimed in claim 4 or 5 wherein the nucleic acid is single stranded.

7. A conjugate probe as claimed in claim 4, 5 or 6 having a labelling group to nucleic acid mass ratio from 1:2 to 15:1, for example from 2:1 to 10:1 and preferably from 4:1 to 8:1.

8. A conjugate probe as claimed in any one of claims 1 to 7, wherein the labelling group is an antibody, a zymogen activator, an antigen, a coloured or fluorescent dye, an enzyme, a chemiluminescent catalyst, a phycobilin, ferritin, a nucleic acid binding protein or a radiolabelled molecule.

9. A conjugate probe as claimed in any one of claims 1 to 8, wherein the labelling group is a molecule which is detectable directly or indirectly.

10. A water-soluble conjugate probe composition comprising
    (1) a water-soluble conjugate probe as claimed in any one of claims 1 to 9; and
    (2) water.

11. A composition as claimed in claim 10 further comprising a cationic detergent.

**12.** A composition as claimed in claim 10 or 11 further comprising glutaraldehyde.

**13.** A method of making a water-soluble conjugate probe which comprises incubating a macromolecule, a labelling group and an aqueous solution of polyglutaraldehyde and recovering said aqueous soluble conjugate probe.

**14.** A method as claimed in claim 13 wherein the aqueous solution comprises a mixture of polyglutaraldehyde and monoglutaraldehyde.

**15.** A method as claimed in claim 13 or 14 which further comprises incubating a cationic detergent and/or a bridging reagent.

**16.** A method as claimed in any one of claims 13 to 15, wherein either: the macromolecule is first incubated with polyglutaraldehyde to form an activated macromolecule and the activated macromolecule is then incubated with the labelling group; or the labelling group is first incubated with polyglutaraldehyde to form an activated labelling group and the activated labelling group is then incubated with the macromolecule.

**17.** A method as claimed in any one of claims 13 to 16 wherein said macromolecule is a protein, a nucleic acid or a receptor target molecule.

**18.** A method as claimed in any one of claims 13 to 17 wherein the reagents, including the macromolecule and labelling group have a final concentration in the incubation mixture of from 0.25mg/ml to 2.0mg/ml, preferably from 1mg/ml to 2mg/ml.

**19.** A hybrid comprising a target molecule bound to a complementary conjugate probe as claimed in any one of claims 4 to 9.

**20.** A method of detecting the presence of a target molecule which comprises:
(a) making a complex by a method comprising placing a target molecule in close proximity to a complementary or specifically binding conjugate probe as claimed in any one of claims 1 to 9, under conditions that will not prohibit binding of said molecule and said probe; and
(b) detecting the presence of the bound labelling group.

**21.** A method according to claim 20 wherein the target is a nucleic acid and the complementary conjugate probe is as defined in any one of claims 4 to 9.

**22.** A method as claimed in claim 20 or 21 wherein unbound labelling group is separated from bound labelling group before detecting the presence of the bound labelling group.

**23.** A test kit for detecting a particular target comprising in a packaged combination:
(1) a conjugate probe as claimed in any one of claims 1 to 9;
(2) a hybridisation medium; and
(3) a denaturation agent.

**24.** A test kit as claimed in claim 23 wherein the concentration of conjugate probe is from 1$\mu$g/ml to 50$\mu$g/ml, preferably from 2$\mu$g/ml to 30$\mu$g/ml.

**25.** A test kit as claimed in claim 23 or 24 further comprising a solid support.

**26.** A test kit as claimed in claim 23, 24 or 25 further comprising a signal generating system.

**27.** A test kit as claimed in any one of claims 23 to 26 for the diagnosis of a virus (such as cytomegalovirus, papilloma virus, herpes simplex virus or Epstein Barr virus), a bacteria, a parasite, a fungus, a genetic disease or a genetic propensity for a disease.

**Patentansprüche**

**1.** Wasserlösliche Konjugatsonde, umfassend:
(a) ein Makromolekül, konjugiert mit

EP 0 401 313 B1

(b) einer Markierungsgruppe mittels
(c) Polyglutaraldehyd.

2. Wasserlösliche Konjugatsonde nach Anspruch 1, weiters umfassend ein brückenbildendes Reagens.

3. Konjugatsonde nach Anspruch 1, wobei das Makromolekül ein Protein (beispielsweise ein Antigen, ein Antikörper, ein Immunglobulin oder ein Rezeptor), eine Nucleinsäure oder ein Rezeptorzielmolekül ist.

4. Wasserlösliche Konjugatsonde, umfassend:
    (a) eine Nucleinsäure, konjugiert mit
    (b) einer Markierungsgruppe mittels
    (c) Polyglutaraldehyd.

5. Konjugatsonde nach Anspruch 4, wobei die Nucleinsäure DNA, RNA oder ein ähnliches Polymer ist.

6. Konjugatsonde nach Anspruch 4 oder 5, wobei die Nucleinsäure einzelsträngig ist.

7. Konjugatsonde nach Anspruch 4, 5 oder 6 mit einem Massenverhältnis von Markierungsgruppe zu Nucleinsäure von 1:2 bis 15:1, beispielsweise von 2:1 bis 10:1 und vorzugsweise von 4:1 bis 8:1.

8. Konjugatsonde nach einem der Ansprüche 1 bis 7, wobei die Markierungsgruppe ein Antikörper, ein Zymogenaktivator, ein Antigen, ein farbiger oder fluoreszierender Farbstoff, ein Enzym, ein chemilumineszenter Katalysator, ein Phycobilin, Ferritin, ein Nucleinsäure bindendes Protein oder ein radioaktiv markiertes Molekül ist.

9. Konjugatsonde nach einem der Ansprüche 1 bis 8, wobei die Markierungsgruppe ein Molekül ist, welche direkt oder indirekt feststellbar ist.

10. Wasserlösliche Konjugatsondenzusammensetzung, umfassend
    (1) eine wasserlösliche Konjugatsonde nach einem der Ansprüche 1 bis 9; und
    (2) Wasser.

11. Zusammensetzung nach Anspruch 10, des weiteren umfassend ein kationisches Detergens.

12. Zusammensetzung nach Anspruch 10 oder 11, weiters umfassend Glutaraldehyd.

13. Verfahren zur Herstellung einer wasserlöslichen Konjugatsonde, welches das Inkubieren eines Makromoleküls, einer Markierungsgruppe und einer wässerigen Lösung aus Polyglutaraldehyd sowie das Rückgewinnen der wässerigen löslichen Konjugatsonde umfaßt.

14. Verfahren nach Anspruch 13, wobei die wässerige Lösung ein Gemisch aus Polyglutaraldehyd und Monoglutaraldehyd umfaßt.

15. Verfahren nach Anspruch 13 oder 14, welches weiters das Inkubieren eines kationischen Detergens und/oder eines brückenbildenden Reagens umfaßt.

16. Verfahren nach einem der Ansprüche 13 bis 15, wobei entweder: das Makromolekül zunächst mit Polyglutaraldehyd inkubiert wird, um ein aktiviertes Makromolekül zu bilden, und das aktivierte Makromolekül daraufhin mit der Markierungsgruppe inkubiert wird; oder die Markierungsgruppe zunächst mit Polyglutaraldehyd inkubiert wird, um eine aktivierte Markierungsgruppe zu bilden, und die aktivierte Markierungsgruppe sodann mit dem Makromolekül inkubiert wird.

17. Verfahren nach einem der Ansprüche 13 bis 16, wobei das Makromolekül ein Protein, eine Nucleinsäure oder ein Rezeptorzielmolekül ist.

18. Verfahren nach einem der Ansprüche 13 bis 17, wobei die Reagentien, welche das Makromolekül und die Markierungsgruppe umfassen, eine Endkonzentration im Inkubationsgemisch von 0,25 mg/ml bis 2,0 mg/ml, vorzugsweise von 1 mg/ml bis 2 mg/ml, aufweisen.

19. Hybrid, umfassend ein Zielmolekül, welches an eine komplementäre Konjugatsonde nach einem der Ansprüche 4 bis 9 gebunden ist.

19

**20.** Verfahren zum Feststellen des Vorhandenseins eines Zielmoleküls, welches umfaßt:
(a) das Herstellen eines Komplexes mittels eines Verfahrens, umfassend das Anordnen eines Zielmoleküls in nächster Nähe einer komplementären oder spezifisch bindenden Konjugatsonde nach einem der Ansprüche 1 bis 9 unter Bedingungen, welche das Binden des Moleküls und der Sonde nicht verhindern; und
(b) das Feststellen des Vorhandenseins der gebundenen Markierungsgruppe.

**21.** Verfahren nach Anspruch 20, wobei das Ziel eine Nucleinsäure ist und die komplementäre Konjugatsonde derart ist, wie in einem der Ansprüche 4 bis 9 beschrieben wird.

**22.** Verfahren nach Anspruch 20 oder 21, wobei die ungebundene Markierungsgruppe von der gebundenen Markierungsgruppe getrennt wird, bevor das Vorhandensein der gebundenen Markierungsgruppe festgestellt wird.

**23.** Analysenset zum Feststellen eines bestimmten Zieles, welcher in einer verpackten Kombination umfaßt:
(1) eine Konjugatsonde nach einem der Ansprüche 1 bis 9;
(2) ein Hybridisierungsmedium; und
(3) ein Denaturierungsmittel.

**24.** Analysenset nach Anspruch 23, wobei die Konzentration der Konjugatsonde zwischen 1 μg/ml und 50 μm/ml, vorzugsweise zwischen 2 μg/ml und 30 μg/ml, beträgt.

**25.** Analysenset nach Anspruch 23 oder 24, das weiters einen festen Träger umfaßt.

**26.** Analysenset nach Anspruch 23, 24 oder 25, das weiters ein Signalerzeugungssystem umfaßt.

**27.** Analysenset nach einem der Ansprüche 23 bis 26 zur Diagnose eines Virus (beispielsweise Zytomegalie-Virus, Papillomavirus, Herpes-simplex-Virus oder Epstein-Barr-Virus), eines Bakteriums, eines Parasiten, eines Pilzes, einer Erbkrankheit oder einer genetischen Veranlagung für eine Krankheit.

**Revendications**

**1.** Sonde conjuguée soluble dans l'eau, comprenant :
(a) une macromolécule conjuguée à
(b) un groupe marqueur par
(c) du polyglutaraldéhyde.

**2.** Sonde conjuguée suivant la revendication 1, comprenant de plus un réactif de pontage.

**3.** Sonde conjuguée suivant la revendication 1, dans laquelle la macromolécule est une protéine (telle qu'un antigène, un anticorps, une immunoglobine ou un récepteur), un acide nucléique ou une molécule cible réceptrice.

**4.** Sonde conjuguée soluble dans l'eau, comprenant :
(a) un acide nucléique conjugué à
(b) un groupe marqueur par
(c) du polyglutaraldéhyde.

**5.** Sonde conjuguée suivant la revendication 4, dans laquelle l'acide nucléique est de l'ADN, de l'ARN ou un polymère similaire.

**6.** Sonde conjuguée suivant la revendication 4 ou 5, dans laquelle l'acide nucléique est à brin simple.

**7.** Sonde conjuguée suivant la revendication 4, 5 ou 6, ayant un rapport pondéral du groupe marqueur à l'acide nucléique de 1:2 à 15:1, par exemple de 2:1 à 10:1, et de préférence, de 4:1 à 8:1.

**8.** Sonde conjuguée suivant l'une quelconque des revendications 1 à 7, dans laquelle le groupe marqueur est un anticorps, un activateur zymogène, un antigène, un colorant visible ou fluorescent, une enzyme, un catalyseur chimioluminescent, une phycobiline, la ferritine, une protéine fixant un acide nucléique ou une molécule radiomarquée.

9. Sonde conjuguée suivant l'une quelconque des revendications 1 à 8, dans laquelle le groupe marqueur est une molécule qui est détectable directement ou indirectement.

10. Composition de sonde conjuguée soluble dans l'eau, comprenant :
    (1) une sonde conjuguée soluble dans l'eau suivant l'une quelconque des revendications 1 à 9, et
    (2) de l'eau.

11. Composition suivant la revendication 10, comprenant de plus, un détergent cationique.

12. Composition suivant la revendication 10 ou 11, comprenant de plus, du glutaraldéhyde.

13. Procédé de préparation d'un sonde conjuguée soluble dans l'eau, qui comprend l'incubation d'une molécule, d'un groupe marqueur et d'une solution aqueuse de polyglutaraldéhyde et la récupération de la sonde conjuguée soluble dans l'eau.

14. Procédé suivant la revendication 13, dans lequel la solution aqueuse comprend un mélange de polyglutaraldéhyde et de monoglutaraldéhyde.

15. Procédé suivant la revendication 13 ou 14, qui comprend, de plus, l'incubation d'un détergent cationique et/ou d'un réactif de pontage.

16. Procédé suivant l'une quelconque des revendications 13 à 15, dans lequel la macromolécule est d'abord incubée avec le polyglutaraldéhyde pour former une macromolécule activée et la molécule activée est ensuite incubée avec le groupe marqueur ou, le groupe marqueur est d'abord incubé avec le polyglutaraldéhyde pour former un groupe marqueur activé et le groupe marqueur activé est ensuite incubé avec la macromolécule.

17. Procédé suivant l'une quelconque des revendications 13 à 16, dans lequel la macromolécule est une protéine, un acide nucléique ou une molécule cible réceptrice.

18. Procédé suivant l'une quelconque des revendications 13 à 17, dans lequel les réactifs, comprenant la macromolécule et le groupe marqueur, ont une concentration finale dans le mélange d'incubation de 0,25 mg par ml à 2,0 mg par ml, de préférence, de 1 mg par ml à 2 mg par ml.

19. Hybride comprenant une molécule cible liée à une sonde conjuguée complémentaire suivant l'une quelconque des revendications 4 à 9.

20. Procédé de détection de la présence d'une molécule cible, qui comprend :
    (a) la préparation d'un complexe par un procédé comprenant la mise en présence d'une molécule cible avec une sonde conjuguée à liaison complémentaire ou spécifique, suivant l'une quelconque des revendications 1 à 9, dans des conditions qui n'empêchent pas la liaison de la molécule à la sonde, et
    (b) la détection de la présence du groupe marqueur lié.

21. Procédé suivant la revendication 20, dans lequel la cible est un acide nucléique et la sonde conjuguée complémentaire est définie dans l'une quelconque des revendications 4 à 9.

22. Procédé suivant la revendication 20 ou 21, dans lequel le groupe marqueur non lié est séparé du groupe marqueur lié avant détection de la présence du groupe marqueur lié.

23. Equipement d'épreuve pour détecter une cible particulière, comprenant, dans un assemblage emballé :
    (1) une sonde conjuguée suivant l'une quelconque des revendications 1 à 9;
    (2) un milieu d'hybridation, et
    (3) un agent dénaturant.

24. Equipement d'épreuve suivant la revendication 23, dans lequel la concentration de la sonde conjuguée se situe entre 1 μg par ml et 50 μg par ml, de préférence, entre 2 μg par ml et 30 μg par mi.

25. Equipement d'épreuve suivant la revendication 23 ou 24, comprenant, de plus, un support solide.

26. Equipement d'épreuve suivant la revendication 23, 24 ou 25, comprenant, de plus, un système générant un signal.

27. Equipement d'épreuve suivant l'une quelconque des revendications 23 à 26, pour diagnostiquer un virus (tel qu'un cytomégalovirus, un papillomavirus, le virus de l'herpès simplex ou le virus d'Epstein Barr), une bactérie, un parasite, un champignon, une maladie génétique ou une tendance génétique à une maladie.

FIG. 1

FIG. 2

FIG. 6

FIG. 3

FIG. 4

FIG. 5